# EUROPEAN PATENT APPLICATION

(11) **EP 1 190 827 A1**
(43) Date of publication of application: **27.03.2002**
(21) Application number: 01120571.3
(22) Date of filing: 29.08.2001
(51) Int. Cl.: B29B 17/02, B07C 5/34, B03B 9/06

(54) **Method and apparatus for identifying and marking the type of material of a plastic piece**

(30) Priority: 30.08.2000 JP 2000261921
(71) Applicant: NEC CORPORATION, Tokyo (JP)
(72) Inventor: Matsushima, Takaaki, c/o NEC Engineering, Ltd., Minato-ku, Tokyo (JP); Yokoyama, Sadahiko, Minato-ku, Tokyo (JP)
(74) Representative: Glawe, Delfs, Moll & Partner

(57) **Abstract**

A plastic identifying apparatus, includes a heating unit (4), a measuring unit (12), and a displaying unit (11). The heating unit (4) heats a plastic object (100). The measuring unit (12) measures a temperature of the plastic object (100) to generate a measurement result and outputs a result data indicating the measurement result. The displaying unit (11) displays the result data on the plastic object (100).

## Description

### Background of the Invention

### 1. Field of the Invention

The present invention relates to a display suitable for selecting plastic wastes by kinds, and a plastic identifying apparatus.

### 2. Description of the Related Art

As a technique for selecting plastic wastes by kinds, a selecting method and an identifying apparatus are well known, for example, which are disclosed in Japanese Laid Open Patent Application (JP-A-Heisei, 8-309749).

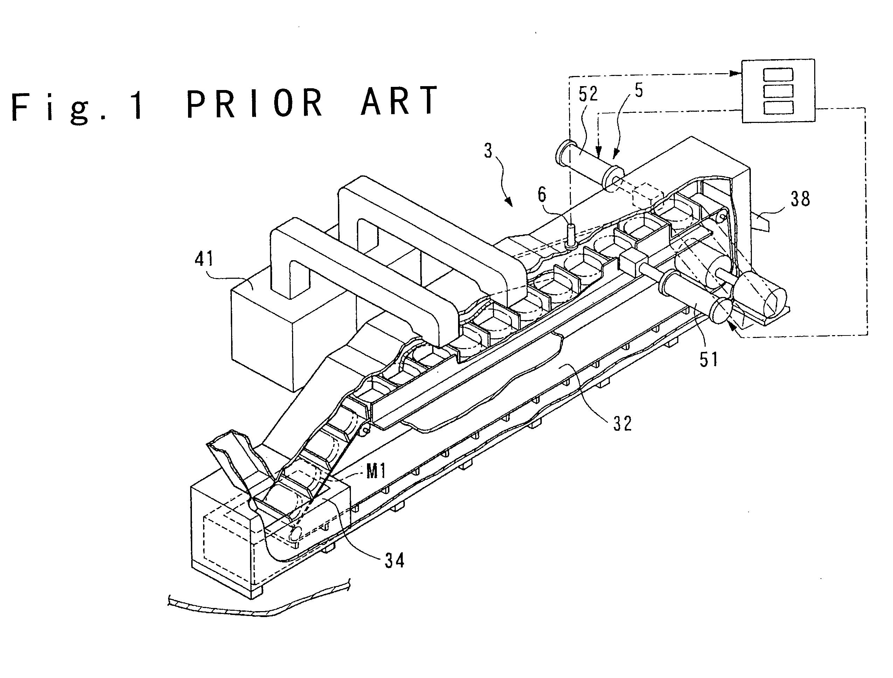

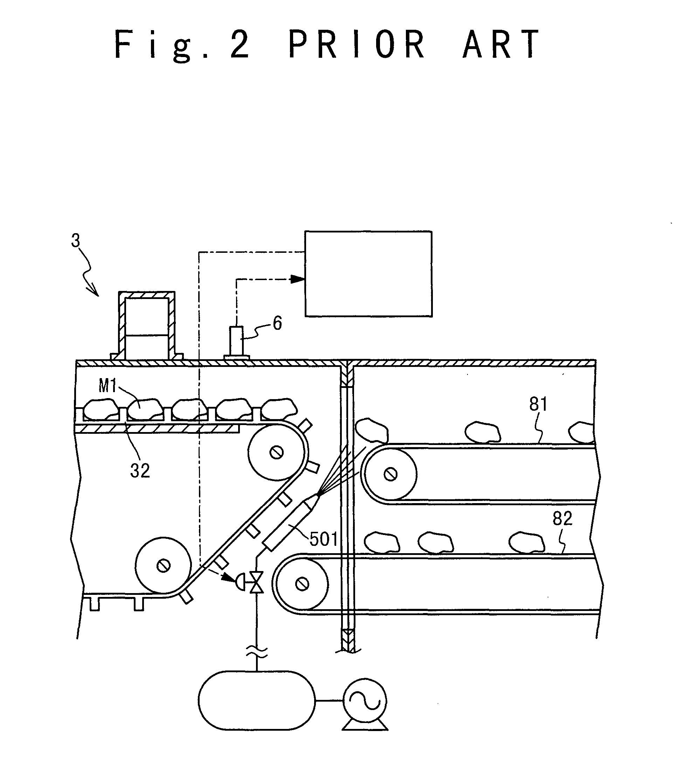

As shown in Figs. 1, 2, this plastic selector is provided with: a crusher (not shown) for crushing the plastic waste into predetermined sizes; a heating furnace (not shown) for preheating the crushed plastic pieces; a micro wave heater 3 for heating a preheated plastic piece M1 by means of a micro wave; a radiation thermometer 6 for measuring a temperature of the plastic piece M1; and a selector 5 for selecting the plastics on the basis of the temperature measurement result of the radiation thermometer 6.

The micro wave heater 3 has a micro wave generator 41 for heating the plastic piece M1 on a conveyer belt 32. The selector 5 has a first cylinder 51, a second cylinder 52 and a third shoot 38 for pushing and discharging the plastic piece M1 from the conveyer belt 32 on the basis of the temperature measurement result of the plastic piece M1 after the heating operation and accordingly selecting PVC.

When the plastic is selected, the crusher for crushing the plastic crushes the plastic into certain sizes. Then, the crushed plastic pieces M1 are preheated by the heating furnace. The preheated plastic pieces M1 are inserted into a hopper 34.

The plastic pieces M1 are carried and moved on the conveyer belt 32 by the clockwise rotation of the conveyer belt 32. The micro waves generated by the micro wave heater 3 are emitted through a wave guide tube towards the moved plastic pieces M1. So, the plastic pieces M1 are further heated.

A surface temperature of the heated plastic pieces M1 is measured by the radiation thermometer 6. The surface temperature is different depending on material. Thus, the material is judged on the basis of the temperature.

When the plastic pieces M1 are further moved and then located at a center of Fig. 2, a spray nozzle is turned ON/OFF on the basis of the above-mentioned temperature data. Accordingly, the plastic pieces M1 are classified into a first conveyer belt 81 and a second conveyer belt 82, respectively.

However, the above-mentioned selection technique has the following problems.
(1) Since there is no idea for a human to identify, a manual selection can not be done. Thus, it is necessary to carry out the selection by using a machine (air from a nozzle). Hence, the apparatus becomes large in size, which makes a cost expensive.
(2) This is the method of spraying the air from the nozzle and then classifying. So, it is difficult to select the pieces selected under a non-aligned condition. Thus, the pieces needs to be fed on the conveyer or the like at a constant interval. Hence, a large number of selection pieces can not be selected at a high speed.
(3) This is the method of spraying the air from the nozzle and then classifying. So, it is necessary to make the sizes of the selection pieces constant. Thus, the requirement of the apparatus such as the crusher causes the apparatus to be large and also causes the cost to be expensive.
(4) This is the method of spraying the air from the nozzle and then classifying. So, it is impossible to select a heavy solid piece that can not be blown away by the air.
(5) The dedicated conveyer and the like are necessary. So, it is impossible to simply install on an existing selection line. Thus, it is necessary to establish a new line. Also, a wide installation area is required for the sake of introduction.
(6) A mark and the like can not be used for the selection piece. Thus, the check can not be done after the selection.

Japanese Laid Open Patent Application (JP-A-Heisei, 5-196587) discloses the following method of determining a kind of resin. This method of determining a kind of resin is a method of determining kinds of a plurality of kinds of resins. It heats a resin product, and then monitors a presence or absence of thermal deformation, and accordingly determines the kind of the resin on the basis of a temperature of an occurrence of the thermal deformation.

Japanese Laid Open Patent Application (JP-A-Heisei, 6-229957) discloses the following material determining method. In this material determining method, a unit for adding a heat to a surface of a determination target sample for a certain period or a unit for removing a heat from a surface of a determination target sample for a certain period heats or cools the surface of the determination target sample. Then, a surface temperature detector detects the surface temperature of the determination target sample heated by the heating unit or cooled by the cooling unit. Accordingly, the material of the determination target sample is determined on the basis of the change property of the surface temperature of the determination target sample obtained by the surface temperature detector.

Japanese Laid Open Patent Application (JP-A-Heisei, 9-262556) discloses the following method of selecting a plastic material. In this method of selecting a plastic material, a micro wave is radiated to a plastic chip in which plastic materials whose kinds are different from each other are mixed, for a certain period. Then, the difference between calorific values caused by a difference between micro wave absorption properties that are different depending on the kinds of the plastics is used to specify a kind of the plastic chip. Accordingly, the plastic chip is selected for each plastic kind.

Japanese Laid Open Patent Application (JP-A-Heisei, 9-297114) discloses the following method of determining a plastic material. In this method of determining a plastic material, infrared is radiated to plastic whose material is not known. Consequently, a surface temperature of the plastic is increased. Then, its temperature is measured to accordingly determine the material.

### Summary of the Invention

The present invention is accomplished in view of the above mentioned problems. Therefore, an object of the present invention is to provide a display on which a measurement result can be known only by viewing a measurement piece, and an apparatus for identifying a plastic material by using the display, and to accordingly make a selection simpler, cheaper and surer.

In order to achieve an aspect of the present invention, a display apparatus, includes: a measuring unit measuring a temperature of an object to generate a measurement result and outputting a picture data indicating the measurement result; and a projecting unit projecting the picture data on the object.

In this case, the measuring unit is a thermography.

In order to achieve another aspect of the present invention, a plastic identifying apparatus, includes: a heating unit heating a plastic object; a measuring unit measuring a temperature of the plastic object to generate a measurement result and outputting a result data indicating the measurement result; and a displaying unit displaying the result data on the plastic object.

In this case, the heating unit heats the plastic object such that the temperature of a surface of the plastic object is different from each material of the plastic object.

Also in this case, the result data indicates a material of the plastic object.

Further in this case, the measuring unit outputs a picture data as the result data, and wherein the displaying unit projects the picture data on the plastic object.

In this case, the measuring unit is a thermography.

In those configurations, the addition of a temperature picture data to a measurement sample/plastic material enables the material difference of the measurement sample/plastic material to be visualized. Thus, the manual selection can be easily done.

In this case, the displaying unit marks a marking corresponding to the result data on the plastic object.

Also in this case, the displaying unit colors a coloring material corresponding to the result data on the plastic object.

In this configuration, since an identification mark classified on the basis of a measurement temperature is added to a plastic material, the difference of the plastic material can be visualized to easily carry out the manual selection.

Further in this case, a plurality of the plastic objects are supplied continuously to be heated by the heating unit, and measured by the measuring unit and displayed by the displaying unit.

In this case, a measurement range measured by the measuring unit and a display range displayed by the displaying unit overlap one another.

In order to achieve still another aspect of the present invention, a display method, includes: measuring a temperature of an object to generate a measurement result; outputting a picture data indicating the measurement result; and projecting the picture data on the object.

In this case, the measuring and the outputting are performed by a thermography.

In order to achieve yet still another aspect of the present invention, a plastic identifying method, includes: heating a plastic object; measuring a temperature of the plastic object to generate a measurement result; outputting a result data indicating the measurement result; and displaying the result data on the plastic object.

In this case, the heating includes heating the plastic object such that the temperature of a surface of the plastic object is different from each material of the plastic object.

Also in this case, the result data indicates a material of the plastic object.

Further in this case, the outputting includes outputting a picture data as the result data, and wherein the displaying includes projecting the picture data on the plastic object.

In this case, the measuring and the displaying are performed by a thermography.

Also in this case, the displaying includes marking a marking corresponding to the result data on the plastic object.

Further in this case, the displaying includes coloring a coloring material corresponding to the result data on the plastic object.

In this case, a plurality of the plastic objects are supplied continuously to be heated, and measured, and outputted and displayed.

Also in this case, a measurement range where the measuring is performed and a display range where the displaying is performed overlap one another.

In order to achieve yet another aspect of the present invention, a plastic identifying method, includes: heating a plastic object; tactually detecting a temperature of the plastic object to detect a material of the plastic object.

In this configuration, a temperature of a heated plastic material can be tactually detected in a temperature detection region. Thus, it is easy to carry out the manual selection.

### Brief Description of the Drawings

Fig. 1 is a perspective view showing a conventional example of a plastic identifying apparatus;
Fig. 2 is a detailed view showing a selection mechanism (nozzle portion) of the plastic identifying apparatus shown in Fig. 1;
Fig. 3 is a plan view showing a display according to a first embodiment of the present invention and a plastic identifying apparatus including the same;
Fig. 4 is a perspective view of the display and the plastic identifying apparatus shown in Fig. 3;
Fig. 5 is a table showing an experiment result when a rise value of a surface temperature is measured after far-infrared is radiated to plastics whose materials are different from each other; and
Fig. 6 is a plan view showing a plastic identifying apparatus according to a second embodiment of the present invention.

### Description of the Preferred Embodiments

Embodiments of the present invention will be described below with reference to the attached drawings.

### [First Embodiment]

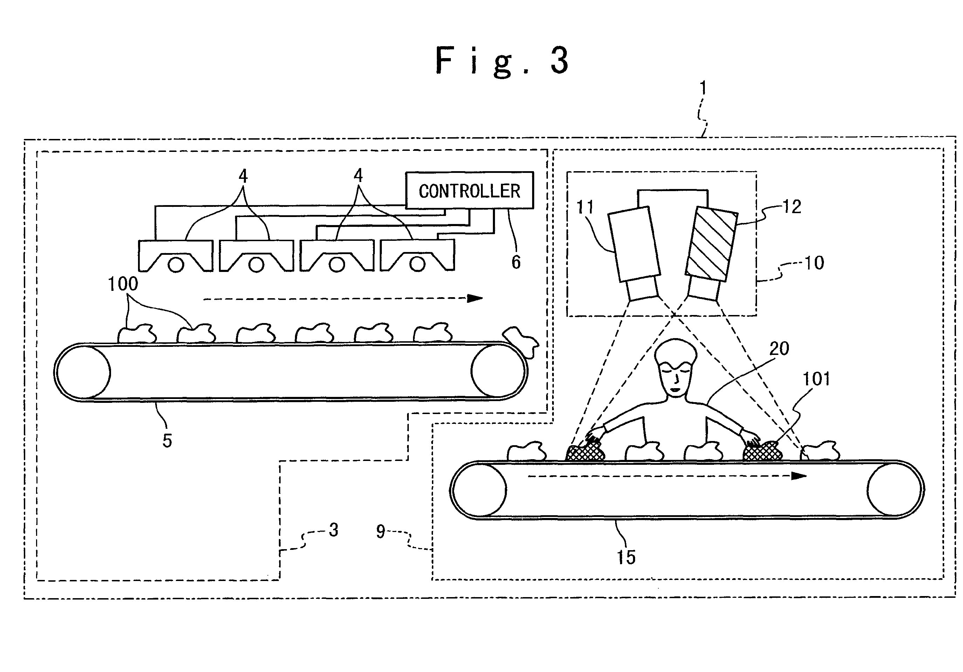

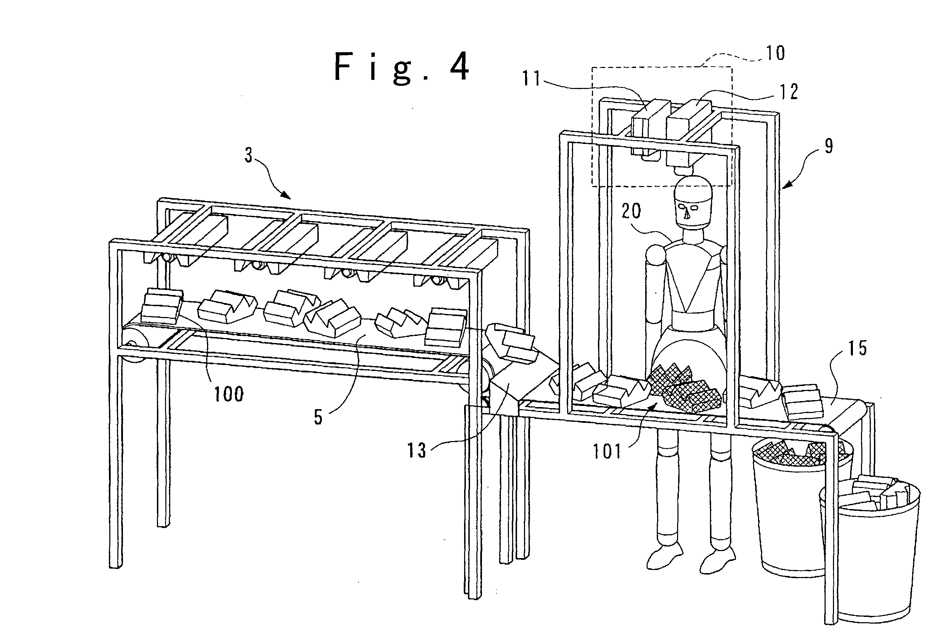

A first embodiment will be described below in detail with reference to Figs. 3, 4. Fig. 3 is a plan view showing a display 10 according to this embodiment and a plastic identifying apparatus 1 having the same.

At first, the configurations of the display 10 and the plastic identifying apparatus 1 are described.

A heater 3 is provided with: a first belt conveyer 5 for conveying a selection plastic material 100; a plurality of heaters (heating units) 4 for heating the plastic material 100 conveyed by this first belt conveyer 5; and a controller 6 for controlling temperatures of those heaters 4 and the like.

The heaters 4 are disposed above the first belt conveyer 5.

An identifying unit 9 is provided with: a second belt conveyer 15 for conveying the selection plastic material 100; and a display 10 for measuring the plastic material 100 and coloring (projecting) it.

The display 10 is located above the second belt conveyer 15 on which the plastic material 100 can be measured/projected.

The display 10 is provided with a thermography (temperature measuring unit) 12 for imaging the measured temperature data; and a projector (picture projector) 11 for projecting the as-is picture pictured by the thermography 12 onto the plastic material 100.

The projector 11 is connected to the thermography 12 through a cable to capture the picture. It is arranged such that the projected range of the picture by the projector 11 is equal to the measurement range of the thermography 12.

The operations of the display 10 and the plastic identifying apparatus 1 will be described below with reference to a perspective view of Fig. 4.

At first, the power supplies for the first belt conveyer 5, the second belt conveyer 15, the heaters 4 and the display 10 are turned on. Next, a temperature of a position to which the plastic material 100 is moved is increased to a certain temperature by the controller 6, and its temperature is kept.

The plastic material 100 is placed on the first belt conveyer 5. Then, while it is moved at a constant pass time (for example, 30 seconds. This time is determined from a length/speed of a belt conveyer), the plastic material 100 is heated.

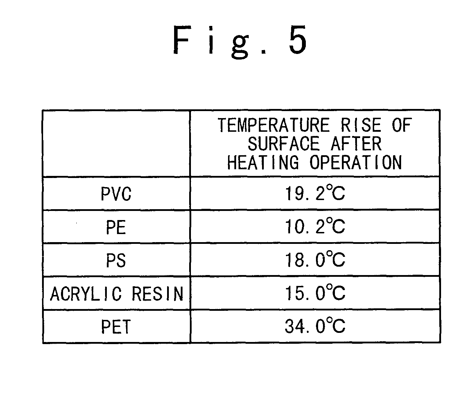

Here, Fig. 5 shows the experiment result when a rise value of a surface temperature is measured after far-infrared is radiated to plastics whose materials are different from each other for 30 seconds. From Fig. 5, it is understood that a temperature rise of a surface is different from each material.

As shown in Fig. 5, the temperature of PVC (PolyVinyl Chloride) is 19.2 °C, and the temperature of PE (Poly-Ethylene) is 10.2 °C, and the temperature of PS (Poly-Styrene) is 18.0 °C, and the temperature of Acrylic resin (PMMA) is 15.0 °C and the temperature of PET (Poly-Ethylene Terephthalate) is 34.0 °C.

After the heating operation, the plastic material 100 is moved to the second belt conveyer 15. At this time, the existence of a slide 13 enables the plastic material 100 to be moved from the first belt conveyer 5 to the second belt conveyer 15 without any turnover.

The second belt conveyer 15, since it is separated from the heater 4, is never heated while the plastic material 100 is moved to the second belt conveyer 15.

When the plastic material 100 goes into the measurement range of the thermography 12/projection range of the projector 11, the difference of the color caused by the temperature difference pictured by the thermography 12 is projected onto the plastic material 100 by the projector 11.

At this time, the measurement range and the projection range are unified. Moreover, the thermography 12 can carry out the measurement at real time. Thus, color information is properly projected onto the plastic material 100 during the movement.

Hence, a selecting person 20 can select a plastic material 101 easily and surely while identifying the color projected onto the plastic material 101.

As mentioned above, the display 10 and the plastic identifying apparatus 1 according to this embodiment enable the plastic material 100 to be manually selected by handpicking. Thus, it is not necessary to carry out the selection by using the machine (the air from the nozzle). Hence, it is possible to miniaturize the apparatus and reduce the cost.

The manual selection by handpicking can be attained to thereby select the non-aligned selection piece that can not be done by the conventional method of spraying the air from the nozzle and then classifying. As a result, it is possible to select the plastic material 100 having any size, shape and weight. Thus, this is very practical. Also, the plastic material 100 can be conveyed in the non-aligned condition. Hence, a large quantity of plastic can be selected at a high speed.

Moreover, the heaters 4 can be placed above the existing first belt conveyer 5. Thus, they can be additionally placed on the existing line to select the plastic material.

So, the dedicated space is not especially required. Thus, it is possible to largely reduce the cost and save the space with regard to the installation.

Moreover, the plastic crusher and the plastic selection mechanism are not required. Hence, it is possible to largely reduce the cost and simplify the selection work.

### [Second Embodiment]

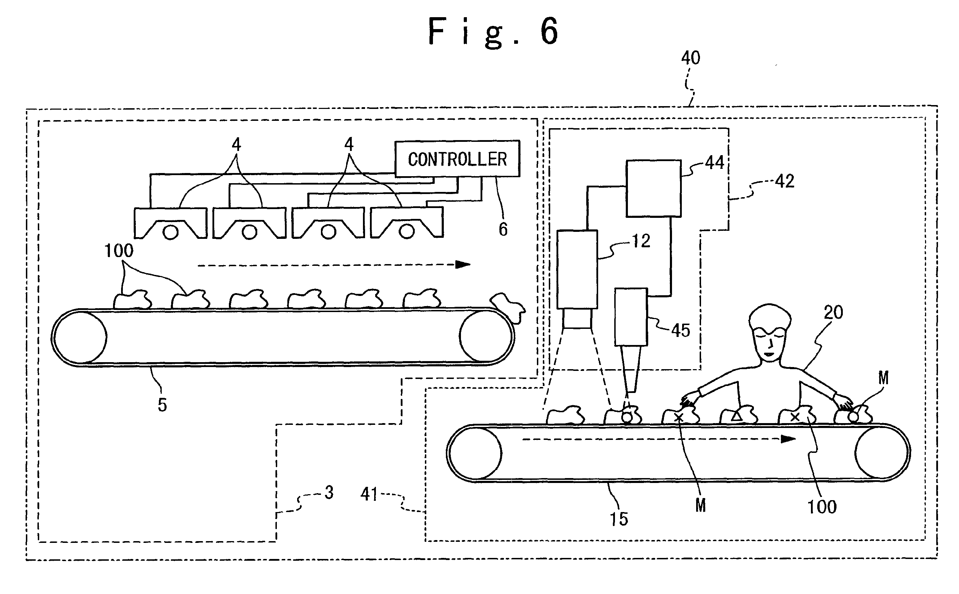

Fig. 6 is a plan view showing a plastic identifying apparatus 40 according to a second embodiment.

The configuration of the plastic identifying apparatus 40 will be described below. However, the configuration of the heater 3 is identical to that of the first embodiment. So, the same symbol equal to that of Fig. 3 is given, and the explanation is omitted. Moreover, as for a selector 41, the same symbols are given to the members equal to those of the first embodiment.

The identifying unit 41 is provided with: the second belt conveyer 15 for conveying the selection plastic material 100; and a painting unit 42 for measuring the plastic material 100 and painting a color and a mark.

The painting unit 42 is placed above the second belt conveyer 15 on which the plastic material 100 can be measured/painted.

The painting unit 42 is composed of: the thermography 12 for imaging the temperature data; a controller 44 for analyzing the picture of the thermography 12; and an ink jet (marking unit) 45 for sticking an identification mark M, such as a mark, a color and the like, on the plastic material 100, in accordance with a command from the controller 44.

As for the identification mark M, a mark different for each material is assigned in advance, on the basis of the relation between the material and the rise temperature after the heating operation, for example, as shown in Fig. 5.

The controller 44 is connected to the thermography 12 through a cable to capture a picture. The ink jet 45 is connected to the controller 44 through a cable to receive a control signal.

The operations of the plastic identifying apparatus 41 will be described below.

By the way, the operations until the plastic material 100 is moved to the second belt conveyer 15 after the power supplies of the first belt conveyer 5, the second belt conveyer 15, the heaters 4 and the display 10 are turned on are identical to those of the first embodiment. Here, their explanations are omitted.

When the plastic material 100 goes into the measurement range of the thermography 12, the controller 44 analyzes the temperature picture data, and determines a spray timing of the ink jet 45.

Then, it instructs the ink jet 45 to spray, on the basis of the analyzed temperature data. Thus, the identification mark M, such as the mark/color and the like, which is different for each material is stuck on the plastic material 100.

Accordingly, the selecting person 20, while identifying the mark/color stuck on the plastic material 100, can select the plastic material 100 easily and surely.

As mentioned above, according to the plastic identifying apparatus 41 in this embodiment, the following effect can be obtained in addition to the effects obtained in the first embodiment.

That is, the mark/color serving as the identification mark M is stuck on the selected plastic material 100. Thus, even if a selection miss occurs on a selection line, it can be later confirmed.

Hence, the selection of the plastic can be done further sure and accurate.

By the way, the present invention is not limited to the above-mentioned embodiments. Also, the above-mentioned respective actual values are only examples. They are not limited to them.

For example, in the above-mentioned embodiments, the thermography 12 is used as the temperature measuring unit. However, for example, an infrared camera and the like may be used. Also, the heater 4 is used as the unit for increasing the temperature of the plastic material 100. However, for example, a unit for electrifying the plastic material 100 may be used.

In the above-mentioned embodiments, instead of the display 10 and the painting unit 42, a temperature detection region is defined to tactually detect the temperature of the heated plastic material 100, and the temperature of the plastic material 100 is increased by the heating operation, the electrifying operation and the like. Then, the selecting person 20 touches with his or her hand the various plastic materials 100 having different temperatures caused by the materials, in the temperature detection region. Accordingly, the difference of the material can be detected to easily carry out the manual selection.

As mentioned above, according to the present invention, it is possible to obtain the following effects.
(1) The manual selection of the plastic material can be attained. Thus, it is not necessary to carry out the selection by using the machine (the air from the nozzle).
   Hence, it is possible to attain the miniaturization of the apparatus and the reduction in the cost.
(2) The manual selection can be attained to thereby select the non-aligned selection piece that can not be done by the conventional method of spraying the air from the nozzle and then classifying. As a result, it is possible to select the plastic material having any size, shape and weight. Thus, this is very practical.
   Also, the plastic material can be conveyed in the non-aligned condition. Hence, a large quantity of plastic material can be selected at a high speed.
(3) The heating unit can be placed above the existing belt conveyer. Thus, it can be additionally placed on the existing line to select the plastic material.
   Hence, the dedicated space is not necessary. Thus, it is possible to largely reduce the cost and save the space with regard to the installation.
(4) The identification mark, such as the mark/color and the like is stuck on the selected plastic material. Thus, even if the selection miss occurs on the selection line, it can be later confirmed.

Hence, the selection of the plastic can be done further sure and accurate.

## Claims

1. A plastic identifying apparatus, comprising:
a heating unit (4) heating a plastic object (100);
a measuring unit (12) measuring a temperature of said plastic object (100) to generate a measurement result and outputting a result data indicating said measurement result; and
a displaying unit (11) displaying said result data on said plastic object (100).

2. The plastic identifying apparatus according to Claim 1, wherein said heating unit (4) heats said plastic object (100) such that said temperature of a surface of said plastic object (100) is different from each material of said plastic object (100).

3. The plastic identifying apparatus according to Claim 2, wherein said result data indicates a material of said plastic object (100).

4. The plastic identifying apparatus according to Claim 1, wherein said measuring unit (12) outputs a picture data as said result data, and
wherein said displaying unit (11) projects said picture data on said plastic object (100).

5. The plastic identifying apparatus according to Claim 4, wherein said measuring unit (12) is a thermography (12).

6. The plastic identifying apparatus according to Claim 1, wherein said displaying unit (45) marks a marking corresponding to said result data on said plastic object (100).

7. The plastic identifying apparatus according to Claim 1, wherein said displaying unit (45) colors a coloring material corresponding to said result data on said plastic object (100).

8. The plastic identifying apparatus according to Claim 1, wherein a plurality of said plastic objects (100) are supplied continuously to be heated by said heating unit (4), and measured by said measuring unit (12) and displayed by said displaying unit (11).

9. The plastic identifying apparatus according to Claim 8, wherein a measurement range measured by said measuring unit (12) and a display range displayed by said displaying unit (11) overlap one another.

10. A plastic identifying method, comprising:
heating a plastic object (100);
measuring a temperature of said plastic object (100) to generate a measurement result;
outputting a result data indicating said measurement result; and
displaying said result data on said plastic object (100).

11. The plastic identifying method according to Claim 10, wherein said heating includes heating said plastic object (100) such that said temperature of a surface of said plastic object (100) is different from each material of said plastic object (100).

12. The plastic identifying method according to Claim 11, wherein said result data indicates a material of said plastic object (100).

13. The plastic identifying method according to Claim 10, wherein said outputting includes outputting a picture data as said result data, and
wherein said displaying includes projecting said picture data on said plastic object (100).

14. The plastic identifying method according to Claim 13, wherein said measuring and said displaying are performed by a thermography (12).

15. The plastic identifying method according to Claim 10, wherein said displaying includes marking a marking corresponding to said result data on said plastic object (100).

16. The plastic identifying method according to Claim 10, wherein said displaying includes coloring a coloring material corresponding to said result data on said plastic object (100).

17. The plastic identifying method according to Claim 10, wherein a plurality of said plastic objects (100) are supplied continuously to be heated, and measured, and outputted and displayed.

18. The plastic identifying method according to Claim 17, wherein a measurement range where said measuring is performed and a display range where said displaying is performed overlap one another.
